# FASCICULE DE BREVET EUROPEEN

(11) **EP 0 920 301 B1**
(45) Date de publication et mention de la délivrance du brevet: **03.09.2003**
(21) Numéro de dépôt: 98929540.7
(22) Date de dépôt: 10.06.1998
(51) Int. Cl.: A61K 9/00

(54) **COMPOSITION THERAPEUTIQUE OU HYGIENIQUE A LIBERATION CONTROLEE ET PROLONGEE DE PRINCIPE(S) ACTIF(S), NOTAMMENT A USAGE OPHTALMIQUE**
THERAPEUTISCHE ODER HYGIENISCHE ZUSAMMENSETZUNG MIT KONTROLLIERTER UND VERLÄNGERTER FREISETZUNG EINER AKTIVEN SUBSTANZ, BESONDERS ZUR VERWENDUNG IN DER AUGENHEILKUNDE
THERAPEUTIC OR HYGIENIC COMPOSITIONS WITH CONTROLLED AND PROLONGED RELEASE OF THE ACTIVE PRINCIPLE(S), IN PARTICULAR FOR OPHTHALMIC USE

(30) Priorité: 11.06.1997 FR 9707231
(43) Date de publication de la demande: 09.06.1999
(73) Titulaire: VETOQUINOL S.A., 70204 Lure Cedex (FR)
(72) Inventeur: GURNY, Robert, CH-1204 Genève (CH); KALTSATOS, Vassilios, F-33500 Libourne (FR); BAEYENS, Vincent, B-4960 Malmédy (BE); BOISRAME, Bernard, F-70200 Lure (FR)
(74) Mandataire: Kédinger, Jean-Paul
(86) Numéro de dépôt international: FR9801191
(87) Numéro de publication internationale: WO98056346

(56) Documents cités:
- FLORIAN GURTLER, ET AL.: "Long-acting soluble Bioadhesive Ophthalmic Drug Insert (BODI) containing gentamicin for veterinary use: optimization and clinical investigation" JOURNAL OF CONTROLLED RELEASE, vol. 33, no. 2, février 1995, pages 231-236, XP000488320 AMSTERDAM, NL
- M. A. ATTIA: "In vivo performance or [3H]dexamethasone ophthalmic film delivery systems in the rabbit eye" INTERNATIONAL JOURNAL OF PHARMACEUTICS, vol. 47, no. 1-3, 1988, pages 21-30, XP002050798
- F. GURTLER, ET AL.: "Keeping the drug in your eye" CHEMTECH, vol. 25, no. 4, avril 1995, pages 33-34, XP002050800

## Description

La présente invention a pour objet une composition thérapeutique ou hygiénique, préparée par extrusion ou thermoformage, à usage externe, notamment à usage ophtalmique en tant qu'insert ou pour application sur la muqueuse buccale ou nasale, ou à usage interne, notamment pour administration orale, cette composition permettant une libération prolongée et contrôlée des principes actifs qu'elle contient ; elle a en outre pour objet un procédé de préparation d'une telle composition.

Dans le domaine ophtalmologique, la plupart des médicaments sont souvent appliqués sous forme de collyres. Cependant, l'un des problèmes essentiels de ces derniers réside dans le fait que l'administration du médicament est "pulsé" en ce sens que pendant une période initiale, il y a surdosage du médicament, laquelle période initiale est suivie d'une période plus longue de dosage insuffisant du médicament. Il s'ensuit que de fréquentes instillations de collyre peuvent être nécessaires pour atteindre un niveau thérapeutique, avec tous les inconvénients que ceci comporte pour le sujet traité.

La thérapie oculaire s'est de ce fait orientée vers la mise au point d'inserts ophtalmiques comprenant un matériau hydrosoluble contenant au moins un principe actif, ces inserts étant destinés à être déposés soit dans le cul-de-sac conjonctival, soit sous la paupière supérieure. L'emploi d'inserts est notamment recherché lorsque le traitement thérapeutique prescrit doit être de longue durée. Le matériau constituant ces inserts est alors choisi pour assurer aussi bien des temps de séjour prolongés de l'insert dans le cul-de-sac conjonctival ou sous la paupière, qu'une libération appropriée et durable du principe actif (voir brevet français No. 92 03390).

Il est à noter cependant que selon le but du traitement et/ou la nature des principes actifs mis en oeuvre, une vitesse de libération des principes actifs encore plus faible que celle obtenue avec les matériaux ou insert connus à ce jour peut être souhaitable pour parvenir alors à des durées de libération pouvant atteindre plusieurs jours.

Une composition pour l'administration oculaire du gentamicin offrant une libération prolongée du principe actif a été publiée par GURTLER (GURTLER F. ET AL., JOURNAL OF CONTROLLED RELEASE, vol. 33, no. 2, 1995, pages 231-236; GURTLER F. ET AL, CHEMTECH, vol. 25, no. 4, 1995, pages 33-34).

Par ailleurs, il est des traitements qui requièrent l'utilisation de plusieurs principes actifs, ceux-ci-devant dans certains cas être libérés successivement suivant une séquence déterminée ou, dans d'autres cas, être libérés de manière concomitante.

La présente invention propose un nouveau type d'insert présentant l'avantage de pouvoir répondre aux besoins soulignés ci-dessus.

Ainsi, la présente invention a pour objet une composition thérapeutique ou hygiénique telle que définie au premier paragraphe de cette description, qui comprend plusieurs principes actifs distribués sensiblement uniformément au sein d'une matrice constituée par un matériau extrudable ou thermoformable et hydrosoluble et/ou dégradable et/ou érodable, caractérisée en ce que chaque principe actif se présente sous la forme d'une dispersion solide avec une substance hydrosoluble et/ou dégradable et/ou capable de perdre sa cohésion physique lorsqu'elle est amenée en contact avec un fluide biologique, cette substance étant ni extrudable ni thermoformable dans les conditions de l'extrusion ou du thermoformage.

On ajoutera que dans la présente description et les revendications annexées "un principe actif se présentant sous la forme d'une dispersion solide avec une substance hydrosoluble et/ou dégradable et/ou capable de perdre sa cohésion physique" vise un principe actif particulaire dont chaque particule est pourvue d'un revêtement constitué de ladite substance, ce revêtement diminuant en quelque sorte la solubilité du principe actif.

On comprendra que lorsque la composition selon l'invention est amenée en contact avec un fluide biologique tel que le liquide lacrymal dans le cas d'un insert, la matrice est progressivement dissoute, dégradée ou érodée, ce qui a pour effet de libérer progressivement et de manière contrôlée l'entité constituée par la dispersion solide qui, à son tour, va progressivement libérer le principe actif (par dissolution, dégradation ou perte de cohésion physique, du revêtement entourant chaque particule de principe actif). Ces deux libérations successives confèrent donc à ladite composition une durée de libération globale prolongée par rapport aux compositions de l'état antérieur de la technique dont le principe actif n'est pas sous forme de dispersion solide.

La composition selon l'invention comprend plusieurs principes actifs se présentant chacun sous la forme d'une dispersion solide, les dispersions solides respectives comprenant toutes la même substance hydrosoluble et/ou dégradable et/ou capable de perdre sa cohésion physique ; dans ce cas, on assistera pendant toute la durée d'utilisation de la composition à une libération concomitante de tous les principes actifs.

Selon encore un autre mode de réalisation, la composition selon l'invention comprend plusieurs principes actifs se présentant chacun sous la forme d'une dispersion solide, les substances entrant dans la constitution des dispersions solides respectives étant différentes les unes des autres ; dans ce cas, les durées de libération respectives des différents principes actifs seront fonction des solubilités respectives dans les fluides biologiques desdites substances.

On pourra ainsi grâce à l'invention, réaliser des compositions ayant les profils de libération des principes actifs souhaités en fonction des applications envisagées.

Il est indispensable, selon l'invention, que la substance combinée au principe actif au sein de la dispersion solide ne soit ni extrudable ni thermoformable. En effet, s'il en était autrement, cette dispersion solide serait dissociée au cours de la fabrication de la composition, c'est-à-dire que le revêtement enrobant chaque particule de principe actif serait ramolli puis se mélangerait avec le matériau destiné à former la matrice, avec mise à nu de la particule de principe actif et l'effet recherché de modification de la solubilité de ce dernier par ledit revêtement ne serait pas obtenu.

On rappellera ici que l'extrusion et le thermoformage sont des techniques particulièrement préférées pour préparer des inserts car elles peuvent ne faire intervenir aucun solvant ; en effet, les solvants sont indésirables en technologie pharmaceutique en raison de leur toxicité.

Or, il est tout à fait surprenant de constater que la mise en oeuvre, pour la formation de la dispersion solide, d'une substance non extrudable ou non thermoformable permette malgré tout de préparer la composition selon l'invention par extrusion ou thermoformage ; on s'attendrait en effet à ce que la présence de cette substance non extrudable ou non thermoformable ne permette pas l'utilisation de la technique d'extrusion ou de thermoformage pour préparer la composition.

On ajoutera que selon l'invention, la température d'extrusion ou de thermoformage sera de préférence non supérieure à 160° C pour éviter toute dégradation des constituants de la composition.

Quand ladite ou lesdites substances non extrudables ou non thermoformables sont hydrosolubles, elles sont de préférence choisies dans le groupe constitué par les polymères d'acide acrylique, les polymères d'acide méthacrylique, les esters d'acides carboxyliques avec la cellulose ou des dérivés de la cellulose, les polymères d'esters vinyliques d'acides carboxyliques, les esters d'acides carboxyliques et d 'amidon, et leurs mélanges.

A titre d'exemples, on citera notamment les polymères d'acide méthacrylique connus sous la marque EUDRAGIT L ou S ; l'acétate de cellulose ; l'acétophtalate de cellulose ; l'acétate trimellitate de cellulose ; l'acétobutyrate de cellulose ; l'acétopropionate de cellulose ; le phtalate d'hydroxypropylméthyl cellulose ; l'acétosuccinate d'hydroxypropylméthyl cellulose ; le phtalate acide de méthylcellulose ; le phtalate acide d'éthyl(hydroxyéthyl) cellulose ; le poly(acétophtalate de vinyle) ; l'acétophtalate d'amidon ; et leurs mélanges.

Quand la ou les substances non extrudables ou non thermoformables sont capables de perdre leur cohésion physique lorsqu'elles sont amenées en contact avec un fluide biologique, il peut s'agir de substances subissant une gélification sous l'effet de ce fluide, le ou les principes actifs pouvant ensuite diffuser à travers le gel ainsi formé.

Bien entendu, ladite substance non extrudable ou non thermoformable sera choisie en fonction de la vitesse de libération souhaitée. Il en est de même du rapport en poids du ou des principes actifs à cette substance, ce rapport pouvant notamment varier de 1 à 5, étant précisé, d'une part que la quantité totale de matière non extrudable ou non thermoformable (y compris les principes actifs quand ceux-ci sont eux-mêmes non extrudables ou non thermoformables) de la composition représente de préférence au plus 60 % en poids, et d'autre part que la quantité de ladite substance non extrudable ou non thermoformable plus la quantité des principes actifs non extrudables et non thermoformables représentent au plus 50 % en poids de la composition.

Selon l'invention, la composition comprend le plus généralement de 0,5 à 50 % en poids, par exemple environ 25 % en poids, de principes actifs en fonction de la nature de ces principes actifs, du type d'affection à traiter et de l'état du sujet à traiter.

On ajoutera que lorsque la composition selon l'invention est un insert à usage ophtalmique, il sera de préférence sous la forme de bâtonnet, de disque, de pastille ou de film. Lorsque la composition est à usage interne, par exemple orale, les extrudats de cette composition peuvent être découpés en éléments ou être granulés en vue de leur introduction dans des gélules (auquel cas lesdits éléments ou granulés ont une dimension de préférence inférieure à 500 µm) ou en vue de réaliser des comprimés (auquel cas lesdits éléments ou granulés ont une dimension inférieure à 1000 µm).

A titre de principes actifs, on pourra utiliser les produits les plus divers, tels que par exemple des agents antibactériens, antiviraux, antimycotiques, antiinflammatoires, etc...

Quant à la matrice constituant l'un des éléments de la composition selon l'invention, elle doit être constituée par un matériau qui a la propriété d'être extrudable ou thermoformable et hydrosoluble et/ou biodégradable et/ou bioérodable.

Ce matériau peut être un polymère, de préférence en mélange avec un polymère bioadhésif ; ce polymère est par exemple une hydroxylalkylcellulose, une maltodextrine, un chitosan, un amidon modifié ou un alcool polyvinylique ; quant au polymère bioadhésif, il peut par exemple être constitué par un polymère carboxy vinylique (famille des Carbopol, notamment Carbopol 934P, 980, 984 ou 954) ou une carboxyméthyl cellulose sodique [comme la Blanose ® type 7 ou 9 (de Aqualon) ou la Tylose® C (de Hoechst)].

L'hydroxyalkylcellulose est de préférence une hydroxyéthylcellulose ou une hydroxypropylcellulose de poids moléculaire compris entre 10 000 et 1 000 000, en particulier entre 80 000 et 125 000.

L'amidon modifié est par exemple un amidon prégélatinisé, destructuré ou partiellement hydrolysé.

On trouvera toutes informations utiles sur cette matrice dans le brevet français No. 92 03390 susmentionné.

La présente invention comprend par ailleurs un procédé de préparation de la composition sus-visée, ce procédé étant caractérisé en ce qu'il comprend la préparation de plusieurs dispersions solides chacune entre une partie au moins d'un principe actif et une substance hydrosoluble et/ou dégradable et/ou capable de perdre sa cohésion physique lorsqu'elle est amenée en contact avec un fluide biologique, cette substance étant ni extrudable ni thermoformable dans les conditions de l'extrusion ou du thermoformage ci-après, le mélange de cette ou ces dispersions solides avec un matériau extrudable ou thermoformable et hydrosoluble et/ou dégradable et/ou érodable et l'extrusion ou le thermoformage du mélange résultant.

On notera que la préparation de chaque dispersion solide comprend par exemple la dissolution de ladite substance dans un solvant approprié pour obtenir une solution, le mélange de cette solution avec le principe actif, l'élimination du solvant pour obtenir un résidu solide et le traitement de ce dernier pour l'amener à la granulométrie souhaitée.

L'invention est illustrée à l'aide des exemples ci-après.

### Exemple de référence 1 : Préparation d'inserts ophtalmiques à base de sulfate de gentamicine sous forme de dispersion solide avec l'acétophtalate de cellulose

(a) On dissout à reflux 90 g d'acétophtalate de cellulose dans 50 ml d'acétone. A la solution obtenue, on ajoute, sous agitation, 150 g de sulfate de gentamicine, puis on élimine l'acétone par évaporation sous vide et ensuite dans une étuve sous vide. Le résidu solide est broyé et le résidu broyé est calibré pour ne retenir que les particules d'une granulométrie de 500 µm environ. Ces particules constituent la dispersion solide de la composition selon l'invention.
On notera que cette dispersion solide peut être préparée par d'autres techniques telles que la granulation ou l'enrobage du sulfate de gentamicine à l'aide d'appareils du type "granulateur par pulvérisation".
(b) On mélange dans un mélangeur de l'hydroxypropylcellulose (Klucel ® HXF NF-Aqualon), de l'éthylcellulose (ECN-50 NF® - Hercules) et du Carbopol® 934P - Goodrich respectivement dans les proportions suivantes : 67 %, 30 % et 3 %. Ce mélange est destiné à constituer la matrice de la composition selon l'invention. Dans 30 g de ce mélange, on incorpore 20 g de la dispersion solide préparée en (a) et on homogénéise.
(c) On procède ensuite à l'extrusion du mélange homogénéisé obtenu en (b). Utilisation est faite à cet effet d'un dispositif d'extrusion à vis du type BETOL® 1820J travaillant dans les conditions suivantes : température de la zone 1 : 140° C ; température des zones 2 et 3 : 150° C ; diamètre de la filière : 1,5 mm ; température d'extrusion 160° C ; pression : 200 KPa.
On obtient ainsi des inserts d'un diamètre de 1,45 mm, d'une longueur de 5 mm et d'un poids de 25 mg et leur composition (en % en poids) est comme suit :
- hydroxypropylcellulose 40,2
- éthylcellulose 18,0
- Carbopol 1,8
- acétophtalate de cellulose 15,0
- sulfate de gentamicine 25,0

(d) Ces inserts sont ensuite étudiés en ce qui concerne la cinétique de libération du sulfate de gentamicine.
A cet effet, ils sont placés chacun dans le cul-de-sac conjonctival de lapins et 4 µl de liquide lacrymal sont prélevés en tant qu'échantillons à intervalles réguliers et la concentration du sulfate de gentamicine y est déterminée par immunoessai par polarisation par fluorescence.
Les résultats obtenus sont rassemblés dans le tableau 1 ci-après (6 à 12 animaux par groupe testé).

**TABLEAU 1**

| **Temps (heure)** | 6 | 12 | 18 | 24 | 30 | 36 |
|---|---|---|---|---|---|---|
| - Concentration moyenne (µg/ml) | 3767 | 3707 | 3519 | 3433 | 3357 | 3341 |
| - Ecart type (µg/ml) | 395 | 245 | 101 | 59 | 126 | 300 |
| - Temps (heure) | 42 | 48 | 54 | 60 | 66 | 72 |
| - Concentration moyenne (µg/ml) | 3320 | 3339 | 2671 | 1809 | 1108 | 462 |
| - Ecart type (µg/ml) | 142 | 194 | 85 | 111 | 39 | 10 |

Ces résultats montrent que le sulfate de gentamicine est présent dans le liquide lacrymal, en une concentration efficace supérieure à la concentration inhibitrice minimale, pendant 72 heures.

### Exemple de référence 2 : Préparation d'inserts ophtalmiques à base de phosphate de dexaméthasone et de sulfate de gentamicine, ce dernier étant sous forme de dispersion solide avec l'acétophtalate de cellulose

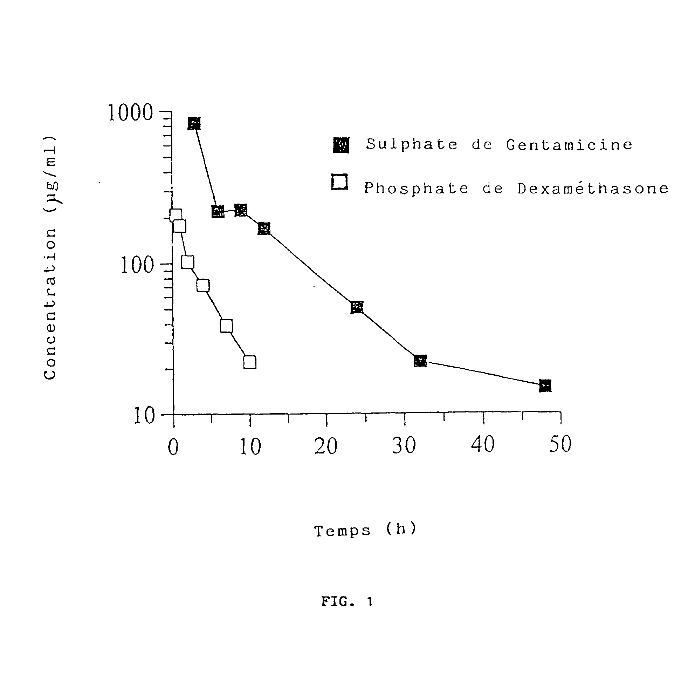

(a) On prépare tout d'abord une dispersion solide du sulfate de gentamicine et de l'acétophtalate de cellulose de la même manière que dans l'exemple 1.
(b) On prépare par ailleurs un mélange d'hydroxypropylcellulose, d'éthylcellulose et de Carbopol, comme à l'exemple 1, puis on y incorpore la dispersion solide préparée en (a) et du phosphate de dexaméthasone. Les quantités respectives des constituants sont choisis pour obtenir la composition suivante :
   - hydroxypropylcellulose 33,2 (% en poids)
   - éthylcellulose 18,0
   - Carbopol 1,8
   - acétophtalate de cellulose 15,0
   - sulfate de gentamicine 25,0
   - phosphate de dexaméthasone 7,0
(c) Le mélange homogène des constituants ci-dessus est ensuite extrudé dans une extrudeuse dans les conditions suivantes : température : 160° C, pression : 300 KPa ; diamètre de filière : 2 mm.
   Les inserts obtenus présentent les caractéristiques suivantes :
   - longueur (5,0 ± 0,2) mm
   - diamètre (2,0 ± 0,05) mm
   - poids (21,0 ± 0,5) g
(d) On procède ensuite à l'évaluation clinique de ces inserts.
   Ces derniers sont, à cet effet, placés chacun dans le sillon conjonctival latéral inférieur de lapins, puis on dose le sulfate de gentamicine et le phosphate de dexaméthasone dans le liquide lacrymal (échantillon de 2 µl prélevés à intervalles réguliers).
   Le dosage du sulfate de gentamicine est réalisé au moyen de l'analyseur TDX® de ABBOTT® LABORATORIES.
   Quant au dosage du phosphate de dexaméthasone, il a été effectué en faisant appel à une technique de microdosage par électrophorèse capillaire.
   Les résultats obtenus sont rassemblés sur la figure unique annexée qui est un graphe donnant la relation entre la concentration (en µg/ml) du sulfate de gentamicine (■) et du phosphate de dexaméthasone (□) dans le liquide lacrymal et le temps.
   Cette figure montre que l'insert selon l'invention permet une libération concomitante du sulfate de gentamicine et du phosphate de dexaméthasone pendant une durée de 10 heures ; ce profil de libération permet au phosphate de dexaméthasone de lutter efficacement contre les dommages susceptibles d'être provoqués par la réponse inflammatoire associée au sulfate de gentamicine. En outre, une concentration de sulfate de gentamicine (supérieure à la concentration inhibitrice minimale de 4 µg/ml) appropriée pour la destruction de la plupart des agents infectieux est assurée pendant 48 heures et ce, grâce au fait que ce sulfate est mis en oeuvre sous la forme d'une dispersion solide avec l'acétophtalate de cellulose, dispersion solide qui modifie (abaisse) en quelque sorte la solubilité dudit sulfate.

## Revendications

1. Composition thérapeutique ou hygiénique, préparée par extrusion ou thermoformage et à usage externe, notamment à usage ophtalmique en tant qu'insert ou pour application sur la muqueuse buccale ou nasale, ou à usage interne, notamment pour administration orale, cette composition comprenant plusieurs principes actifs distribués sensiblement uniformément au sein d'une matrice constituée par un matériau extrudable ou thermoformable et hydrosoluble et/ou dégradable et/ou érodable, **caractérisée en ce que** chaque principe actif se présente sous la forme d'une dispersion solide avec une substance hydrosoluble et/ou dégradable et/ou capable de perdre sa cohésion physique lorsqu'elle est amenée en contact avec un fluide biologique, cette substance étant ni extrudable ni thermoformable dans les conditions de l'extrusion ou du thermoformage.

2. Composition selon la revendication 1, **caractérisée en ce que** les dispersions solides des principes actifs comprennent toutes la même substance hydrosoluble et/ou dégradable et/ou capable de perdre sa cohésion physique.

3. Composition selon la revendication 1, **caractérisée en ce que** les dispersions solides des principes actifs comprennent une substance hydrosoluble et/ou dégradable et/ou apte à perdre sa cohésion, différente d'une dispersion solide à l'autre.

4. Composition selon l'une des revendications 1 à 3, **caractérisée en ce que** ladite ou lesdites substances sont choisies dans le groupe constitué par les polymères d'acide acrylique, les polymères d'acide méthacrylique, les esters d'acides carboxyliques avec la cellulose ou des dérivés de la cellulose, les polymères d'esters vinyliques d'acides carboxyliques, les esters d'acides carboxyliques et d'amidon, et leurs mélanges.

5. Composition selon l'une des revendications précédentes, **caractérisée en ce qu'**une partie au moins desdites substances sont constituées par l'acétophtalate de cellulose.

6. Composition selon l'une des revendications précédentes, **caractérisée en ce que** pour chaque dispersion solide, le rapport en poids du principe actif à la substance est de 10/2 à 10/10.

7. Procédé de préparation de la composition selon l'une des revendications 1 à 6, **caractérisé en ce qu'**il comprend la préparation de dispersions solides chacune entre un principe actif respectif et une substance hydrosoluble et/ou dégradable et/ou capable de perdre sa cohésion physique lorsqu'elle est amenée en contact avec un fluide biologique, cette substance étant ni extrudable ni thermoformable dans les conditions de l'extrusion ou du thermoformage ci-après, le mélange de ces dispersions solides avec un matériau extrudable ou thermoformable et hydrosoluble et/ou dégradable et/ou érodable, et l'extrusion ou le thermoformage du mélange résultant.

8. Procédé selon la revendication 7, **caractérisé en ce que** la préparation de chaque dispersion solide comprend la dissolution de ladite substance dans un solvant approprié pour obtenir une solution, le mélange de cette solution avec le principe actif, l'élimination du solvant pour obtenir un résidu solide et le traitement de ce dernier pour l'amener à la granulométrie souhaitée.

9. Procédé selon la revendication 8, **caractérisé en ce que** ladite substance est l'acétophtalate de cellulose et le solvant est l'acétone.

## Patentansprüche

1. Therapeutische oder hygienische Zusammensetzung, hergestellt durch Extrusion oder Thermoformung und zur äußerlichen Anwendung, insbesondere zur ophthalmischen Anwendung als Einlage oder zur Applikation auf die Wangen- oder Nasenschleimhaut, oder zur innerlichen Anwendung, insbesondere für eine orale Verabreichung, wobei diese Zusammensetzung mehrere Wirkstoffe umfasst, die im Wesentlichen gleichmäßig in einer Matrix verteilt sind, die aus einem extrudierbaren oder thermoformbaren und wasserlöslichen und/oder abbaubaren und/oder erodierbaren Material gebildet ist, **dadurch gekennzeichnet, dass** jeder Wirkstoff in Form einer festen Dispersion mit einer Substanz vorliegt, die wasserlöslich und/oder abbaubar und/oder im Stande ist, ihren physischen Zusammenhalt zu verlieren, wenn sie mit einer biologischen Flüssigkeit in Kontakt gebracht wird, wobei diese Substanz unter den Bedingungen der Extrusion oder der Thermoformung weder extrudierbar noch thermoformbar ist.

2. Zusammensetzung nach Anspruch 1, **dadurch gekennzeichnet, dass** die festen Dispersionen der Wirkstoffe alle die gleiche Substanz umfassen, welche wasserlöslich und/oder abbaubar und/oder im Stande ist, ihren physischen Zusammenhalt zu verlieren.

3. Zusammensetzung nach Anspruch 1, **dadurch gekennzeichnet, dass** die festen Dispersionen der Wirkstoffe eine Substanz umfassen, die wasserlöslich und/oder abbaubar und/oder fähig ist, ihren Zusammenhalt zu verlieren, welche sich von einer festen Dispersion zur anderen unterscheidet.

4. Zusammensetzung nach einem der Ansprüche 1 bis 3, **dadurch gekennzeichnet, dass** die Substanz oder die Substanzen ausgewählt werden aus der Gruppe bestehend aus Polymeren von Acrylsäure, Polymeren von Methacrylsäure, Estern von Carbonsäuren mit Cellulose oder Cellulosederivaten, Polymeren von Vinylestern von Carbonsäuren, Estern von Carbonsäuren und Stärke, und ihren Gemischen.

5. Zusammensetzung nach einem der vorangehenden Ansprüche, **dadurch gekennzeichnet, dass** wenigstens ein Teil der besagten Substanzen aus Celluloseacetophthalat besteht.

6. Zusammensetzung nach einem der vorangehenden Ansprüche, **dadurch gekennzeichnet, dass** für jede feste Dispersion das Gewichtsverhältnis des Wirkstoffs zu der Substanz 10/2 bis 10/10 beträgt.

7. Verfahren zum Herstellen der Zusammensetzung nach einem der Ansprüche 1 bis 6, **dadurch gekennzeichnet, dass** es die Herstellung von festen Dispersionen jeweils zwischen einem entsprechenden Wirkstoff und einer Substanz, die wasserlöslich und/oder abbaubar und/oder im Stande ist, ihren physischen Zusammenhalt zu verlieren, wenn sie mit einer biologischen Flüssigkeit in Kontakt gebracht wird, wobei diese Substanz unter den Bedingungen der nachstehenden Extrusion oder Thermoformung weder extrudierbar noch thermoformbar ist, das Vermischen dieser festen Dispersionen mit einem extrudierbaren oder thermoformbaren und wasserlöslichen und/oder abbaubaren und/oder erodierbaren Material und die Extrusion oder die Thermoformung des resultierenden Gemisches umfasst.

8. Verfahren nach Anspruch 7, **dadurch gekennzeichnet, dass** die Herstellung von jeder festen Dispersion die Auflösung der besagten Substanz in einem geeigneten Lösungsmittel zum Erhalten einer Lösung, das Vermischen dieser Lösung mit dem Wirkstoff, die Entfernung des Lösungsmittels zum Erhalten eines festen Rückstands und die Behandlung des letzteren zum Herbeiführen der gewünschten Körnung umfasst.

9. Verfahren nach Anspruch 8, **dadurch gekennzeichnet, dass** die besagte Substanz Celluloseacetophthalat ist und das Lösungsmittel Aceton ist.

## Claims

1. Therapeutic or hygienic composition, prepared by means of extrusion or thermoforming and for external use, in particular ophthalmic use as an insert, or for application to the oral mucous membrane or to the mucous membrane of the nose, or for internal use, in particular for oral administration, this composition including several active principles distributed substantially uniformly within a matrix constituted by an extrudable or thermoformable and water-soluble and/or degradable and/or erodable material, **characterised in that** each active principle takes the form of a solid dispersion with a substance that is water-soluble and/or degradable and/or capable of losing its physical cohesion when it is brought into contact with a biological fluid, this substance being neither extrudable nor thermoformable under the extrusion or thermoforming conditions.

2. Composition according to claim 1, **characterised in that** the solid dispersions of the active principles all include the same substance that is water-soluble and/or degradable and/or capable of losing its physical cohesion.

3. Composition according to claim 1, **characterised in that** the solid dispersions of the active principles all include a substance that is water-soluble and/or degradable and/or capable of losing its physical cohesion, differing from one solid dispersion to another.

4. Composition according to one of claims 1 to 3, **characterised in that** said substance or said substances are chosen from the group constituted by acrylic acid polymers, methacrylic acid polymers, carboxylic acid esters with cellulose or cellulose derivatives, polymers of vinyl esters of carboxylic acids, esters of carboxylic acids and starch, and mixtures thereof.

5. Composition according to one of the preceding claims, **characterised in that** a part at least of said substances is constituted by cellulose acetophthalate.

6. Composition according to any one of the preceding claims, **characterised in that**, for each solid dispersion, the weight ratio of the active principle to the substance is from 10/2 to 10/10.

7. Process for preparing the composition according to any one of claims 1 to 6, **characterised in that** it includes the preparation of solid dispersions each between a respective active principle and a substance that is water-soluble and/or degradable and/or capable of losing its physical cohesion when it is brought into contact with a biological fluid, this substance being neither extrudable nor thermoformable under the extrusion or thermoforming conditions hereinafter, the mixing of these solid dispersions with a material that is extrudable or thermoformable and water-soluble and/or degradable and/or erodable, and the extrusion or thermoforming of the resulting mixture.

8. Process according to claim 7, **characterised in that** the preparation of each solid dispersion includes dissolving said substance in a suitable solvent to obtain a solution, mixing this solution with the active principle, removing the solvent to obtain a solid residue and treating the latter to give it the desired grain size distribution.

9. Process according to claim 8, **characterised in that** said substance is cellulose acetophthalate and the solvent is acetone.
